# EUROPEAN PATENT APPLICATION

(11) **EP 0 942 283 A2**
(43) Date of publication of application: **15.09.1999**
(21) Application number: 99301897.7
(22) Date of filing: 12.03.1999
(51) Int. Cl.: G01N 33/538, G01N 33/546, G01N 33/58

(54) **Methods and apparatus for improved immunoassay**

(30) Priority: 13.03.1998 US 77895 P
(71) Applicant: Thaco Research Ltd., Chantilly, Virginia 20151 (US)
(72) Inventor: Thacker, James D., Manassas, Virginia 22110 (US); Casale, Ellen S., Ashburn, Virginia 20147 (US)
(74) Representative: Harrison Goddard Foote

(57) **Abstract**

Antigen immobilization and antibody-capture techniques together with microfiltration plates are employed to effect a method for determining the concentration or amount of a test substance present in a sample. A positive dose response results and a wider linear dynamic range is achieved as compared with typical competitive immunoassays. A kit comprising a novel immunoassay is useful and efficient in determining the presence of a test substance in a sample.

## Description

### Background of the Invention

### 1. Field of the Invention

The present invention relates to immunoassays and more particularly to novel competitive immunoassay methods comprising antigen immobilization and antibody-capture useful in conjunction with microfiltration. The invention may he used for detecting an analyte in water, as well as other applications. The present invention also relates to immunoassay systems for high-throughput analysis.

### 2. Description of the Background

An overarching goal of environmental analyses is to assess the risks to human health arising from exposure to hazardous substances ad pathogenic organisms. The Federal Insecticide, Fungicide and Rodenticide Act (FIFRA) was promulgated to assess health risks of new and existing agrochemicals and to regulate their manufacture and use. In so doing, FIFRA purports to address a major source of human exposure to potentially harmful chemical substances.

The adoption of the FIFRA regulatory process has added substantial cost to the development of new agrochemicals, so much so that a typical new product will cost the manufacturer $30 million and require up to seven years to go from discovery to market. A significant portion of the cost and time can be directly attributed to the effort required to perform tens of thousands of analyses using conventional methods. These analytical data packages are required to set, among other things, tolerance levels for the new product. Moreover, the ongoing monitoring requirements imposed by other environmental mandates, such as the Safe Drinking Water Act, add additional after-market costs when conventional methods of analysis are required. Similarly, health, environmental and water quality regulations are being applied in Europe and elsewhere. Both the registration costs and the after-market environmental monitoring costs amount to a significant portion of the total cost for the use of agrochemical products. Clearly a faster and less expensive method of analysis would be a welcome addition to the arsenal of analytical methods.

To develop new methods of analysis that are faster, cheaper and better, it is necessary to fully understand the rationale underlying conventional methods of analysis. The analytical paradigm suggests that detectability of an analyte can be improved by either increasing the selectivity of the detection device, *e.g.* using a mass spectrometer or enhancement of the sensitivity of the detection device, *e.g.* choosing an electrochemical detector vis-à-vis a flame ionization detector. Conventional methods of analysis often employ elaborate sample preparation schemes including chromatography in order to remove interferences and improve sensitivity and overall detectability of the analyte of interest. Sample preparation strategies are a recognition of the fact that even the most selective detectors currently in use are not analyte specific and are subject to both positive and negative interferences.

Immunoassays address the analytical paradigm in two ways. First, the antibody-antigen binding reaction is highly selective. This selectivity reduces and even eliminates the need for elaborate separation procedures to remove interferences. Coupled with the use of ultrasensitive radiochemical, fluorescent, photoluminescent, or visible and ultraviolet markers, immunoassays have made sub-nanogram detection of analytes routine. In spite of the progress made towards reducing the time and cost required for residue analyses, improvements are still needed, particularly in the area of sample preparation. Conventional solid phase ELISA assays can require as many as six hours to complete, due in part to the time constraints for completion of the phase transfer process. In conventional solid phase ELISA assays, typically the first antibody is immobilized in a monolayer on a solid support system to which the test substance is added. This use of an antibody monolayer is limiting to the rate at which antibody can be presented to antigen in order to form antibody-antigen complexes. In addition, conventional ELISA assays are not susceptible to automation. Therefore a need exists for immunoassay methods and systems enabling rapid sample analysis and increased laboratory throughput with a commensurate reduction in analytical costs, and for such methods and systems which are capable of being applied efficiently and accurately.

### Summary of the Invention

The present invention overcomes or mitigates problems and disadvantages associated with current strategies and designs, and provides in preferred embodiments competitive immunoassay methods using techniques of antigen immobilization and antibody-capture, in conjunction with microfiltration plates, to determine the concentration or amount of a test substance in a sample. Positive dose response results for the test substance of interest and a wider linear dynamic range can be achieved using the present invention, as compared with typical competitive immunoassays. The present invention is also directed to immunoassay systems and kits for high-throughput analysis and allows for more rapid sample analysis and increased laboratory throughput with a commensurate reduction in analytical costs.

The invention provides a method for detecting for or measuring an antigen in a sample comprising the steps of:
contacting the sample with a first antibody having binding affinity to the antigen to form antigen-antibody complexes;
contacting uncomplexed antibody with excess antigen immobilized to a solid support to form immobilized antigen-antibody complexes;
capturing unbound antigen-antibody complexes by contacting said antigen-antibody complexes to an immobilized second antibody having binding affinity specific for the first antibody; and
detecting for or measuring antigen in the sample. This final step typically involves quantitating the amount of antigen.

One embodiment of the invention is directed to a method to measure the amount of a substance in a sample, comprising the steps of contacting the sample with a first antibody having binding affinity to an antigen of the substance in order to form free antigen-antibody complexes, contacting remaining uncomplexed first antibody with immobilized antigen to form immobilized antigen-antibody complexes, separating the free antibody-antigen complexes from the immobilized antigen-antibody complexes, contacting the free antigen-antibody complexes to an immobilized second antibody having binding affinity specific for the first antibody in order to capture the free antibody-antigen complexes, labeling the captured antibody-antigen complexes with a detectable label, effecting a measurable change in the label, and measuring the amount of the substance in the sample. The present invention can be used on samples including, but not limited to, a sample of groundwater, tap water, drinking water, food materials, beverages and milk and dairy products. The substances which can be measured or detected include, among others, agrochemicals, pesticides, fungicides, and any other substance hazardous for exposure to humans.

Another embodiment of the invention is directed to a kit useful in detecting for or measuring the amount of a test substance antigen in a sample comprising first antibodies having binding affinity to the test substance antigen, polystyrene beads conjugated with the test substance antigen, a microfilter plate containing the conjugated beads, a capture plate containing immobilized second antibodies, said second antibodies comprising anti-globulin antibodies specific for said first antibody, a label which selectively binds to captured first antibody-antigen complexes, and a substrate which produces a measurable change in the presence of the label.

Other embodiments and advantages of the invention are set forth, in part, in the description which follows, and, in part, will be obvious from this description and may be learned from the practice of the invention.

### Description of the Drawings

Figure 1 is a schematic of one embodiment of a method of the present invention.
Figure 2 is a graph depicting the results of an immunoassay using the method of Figure 1.
Figure 3 is a graph of the results of an assay using a conventional ELISA technique.

### Description of the Invention

As embodied and broadly described herein, the present invention is directed to novel methods for detecting for or measuring (*e.g.* determining the amount of) a test substance in a sample by means of an immunoassay technique.

One embodiment of the present invention is directed to a method for measuring the amount of a test substance or its derivative which may be present in a sample. This method comprises the steps of (A) providing or obtaining a sample comprising an unknown amount of test substance, (B) contacting the sample with first antibodies having binding affinity to an antigen of the test substance to form free antibody-antigen complexes, (C) contacting the remaining uncomplexed first antibody with immobilized antigen to form immobilized antigen-antibody complexes, (D) excluding or separating the free antigen-antibody complexes from the immobilized antigen-antibody complexes and passing these free complexes through a microfilter using means known in the art such as centrifugation or vacuum, (E) contacting the free antigen-antibody complexes with an immobilized second antibody specific for the first antibody, such as an anti-species antibody to capture the free antigen-antibody complexes, (F) labeling the captured antigen-antibody complex with a detectable label, (G) effecting a measurable change in the label and (H) measuring the test substance in the sample.

In a preferred embodiment, the immobilized antigen comprises an antigen coating on polystyrene beads contained in wells of a microfilter plate. The microfilter plate is used to separate the immobilized first antibody-antigen complexes from the free first antibody-antigen complexes. The sample might be ground water, drinking water, tap water or water used for agricultural purposes. The sample might also be a biological sample such as blood, saliva or any other bodily fluid. The sample might also be a food material, a beverage, milk or dairy products. The substance might be a pesticide, a fungicide, a pathogenic organism, a carcinogen or any other substance hazardous for exposure to humans.

The second antibody having binding affinity for the first antibody is preferably a species-specific antibody. It is preferably immobilized on a solid support having at least one surface amenable to attachment of such an antibody. Suitable examples of a solid support include, for example, polystyrene microwell plates, nitrocellulose membranes, latex beads or glass surfaces. In one embodiment, the anti-species or species-specific antibody is an anti-globulin antibody, and is adsorbed onto a polystyrene microwell plate.

In step (C) of the method, immobilized first antibody-antigen complexes are formed between free or excess first antibody and the immobilized antigen, which comprises antigen-coated polystyrene beads coated with the antigen of the substance. As a result, immobilized antigen-antibody complexes and free antigen-antibody complexes are present in the assay. In step (D) the free complexes are separated from the immobilized complexes. Separation may be effected by any suitable means such as centrifugation, vacuum filtration, or a combination of centrifugation and vacuum filtration. and generally includes at least one washing step.

As separation from the immobilized antigen-antibody complex occurs, the free antigen-antibody complexes are captured in step (E) by a second immobilized antibody having affinity for the first antibody. In a preferred embodiment, the first antibody is derived from a first species and the second antibody is derived from a second species. The second antibody is preferably an anti-globulin antibody immobilized on a polystyrene microwell plate. In one preferred embodiment, the second antibody is goat anti-rabbit IgG and the first antibody is rabbit monoclonal antibody.

The captured complexes are labeled with a detectable label in step (F). The label may be any material that has binding affinity to the captured antigen-antibody complex and can be detected. The label may be detected directly or indirectly through interaction with a substrate. Suitable examples of labels include, without limitation, enzymes, colored dyes, fluorescent materials, chemiluminescent materials, bioluminescent materials, and radioactive isotopes. In a preferred embodiment, the detectable label is a enzyme. The enzyme is preferably conjugated with a material having binding affinity for the captured antigen-antibody complex. The binding of the label to the complex may be by, for example, antibody-antigen binding, protein-ligand binding, avidin (streptavidin)-biotin binding, covalent chemical binding or passive adsorption.

In a preferred embodiment, the binding of the label to the captured antibody-antigen complex is by antibody-antigen binding wherein the label is bound to an antibody having binding affinity for the captured antibody-antigen complex. Such binding results in a typical "sandwich" configuration. The antibody bound to the label may be polyclonal or monoclonal. Furthermore, an antibody fragment of any of the above recited antibodies which contains the binding region of the antibody, such as Fab fragments, may be used. The particular antibody conjugated to the label will, of course, depend on the type of antibody bound to the antigen. That is, the antibody conjugated to the label will be chosen to have binding affinity to the immobilized antibody-antigen complex. For example, the first antibody with affinity to the test antigen is produced in rabbits, such as described in the Example below. Consequently, the antibody to which the label is conjugated is preferably an anti-rabbit antibody having binding affinity to the rabbit immunoglobulins.

Once the captured antibody-antigen complex is labeled, a measurable change is effected in the label in step (G) of the method. The measurable change may be effected by irradiation with uv-vis light, fluorescence, electrical waveform, or by introduction of a substrate which interacts with the label to produce a measurable change. It is possible that the measurable change may be inherent in the label. For instance, the label may be a radioactive isotope wherein radioactivity, e.g., beta-rays, may be detected simply by introducing means to measure the radioactivity.

In another embodiment of the invention, the measurable change may be effected by contacting the labeled complex with a substrate. Such substrates are well known in the art and are dependent on the type of label used. In a preferred embodiment, the label is an enzyme and the substrate is a compound capable of interacting with the enzyme, which produce a measurable change. In one embodiment, the enzyme is a phosphatase and the substrate is a phosphate compound. The interaction of the enzyme and the substrate generally results in a compound having a measurable property. For instance, the resulting compound may be fluorescent, luminescent, or strongly uv-absorbent.

Finally, once the measurable change is effected in step (G), the amount or concentration of the test substance is measured or derived in step (H). Such measurement may be by various methods known in the art, such as, for example, uv-vis spectrophotometry, fluorometry, or beta counting, etc.

Another embodiment of the invention is directed to a novel kit for measuring a test substance in a sample comprising (1) immobilized polystyrene beads coated with the specific antigen (test substance antigen) loaded in wells of a microfilter plate, such as a 96-well microfilter plate, (2) first antibody having binding affinity to the test substance antigen, (3) a capture plate coated with immobilized second antibodies, such as a species-specific antibody, having affinity for the first antibody, (4) a label capable of binding to the first antibody-antigen complexes, and (5) a substrate capable of producing a measurable change in the presence of the label. In a preferred embodiment, the antigen of the test substance is a substance hazardous to the health of humans. In one embodiment, the test substance is a biological sample such as a bodily fluid or blood. In this embodiment of the invention, the antigen of the test substance is a biological antigen such as a viral or bacterial antigen, or a non-foreign "self" antigen. In one preferred embodiment, the antigen is an agrochemical and the test substance is a sample of drinking water.

The following example is offered to illustrate embodiments of the invention and should not be viewed as limiting the scope of the invention.

### Example

Wash buffer (PBS and 0.01% Tween 20; "PBST") was prepared from 0.01% Tween-20 in 10 mM phosphate buffered saline (0.145 M NaCl), pH 7.4 (PBS). The first antibodies used were a purified polyclonal culture produced in rabbits by THACO Research, Ltd. to the immunogen, RH2651-KLH.

### Capture Plate Preparation

A 96-well polystyrene ELISA plate (Corning® Easy Wash, Corning Costar Corporation) was coated with goat, anti-rabbit immunoglobulin (Sigma Chemical Co.) at a concentration of 10µg/ml in PBS. The plate thus prepared was sealed with sealing tape, covered and placed in the refrigerator for an overnight incubation at 4°C. Following an overnight incubation, the plate was emptied and washed with PBST to remove unbound immunoglobulin. Unbound binding sites in the wells were blocked with 300 µL bovine serum albumin (BSA) blocking solution (1% BSA in PBS). This solution was incubated at room temperature for at least 10 minutes. The plate thus prepared was used as the "capture plate" in the assay.

### Microfilter Plate Preparation

The wells of a 96-well microfilter plate (0.45 µm Durapore® membrane, Millipore Corporation) were filled with 50-100 µL of pre-washed 90 µm polystyrene beads (Bangs Laboratories). The beads were coated passively *in situ* with coating antigen (RH2651-BSA, THACO Research, Ltd.) at a working concentration of 0.1-1 mg/ml in PBS. The coating solution was removed by vacuum and the beads blocked with BSA blocking solution. After removing the blocking solution, the beads were dried *in vacuo* at room temperature and stored at 4°C prior to use.

### Assay Protocol

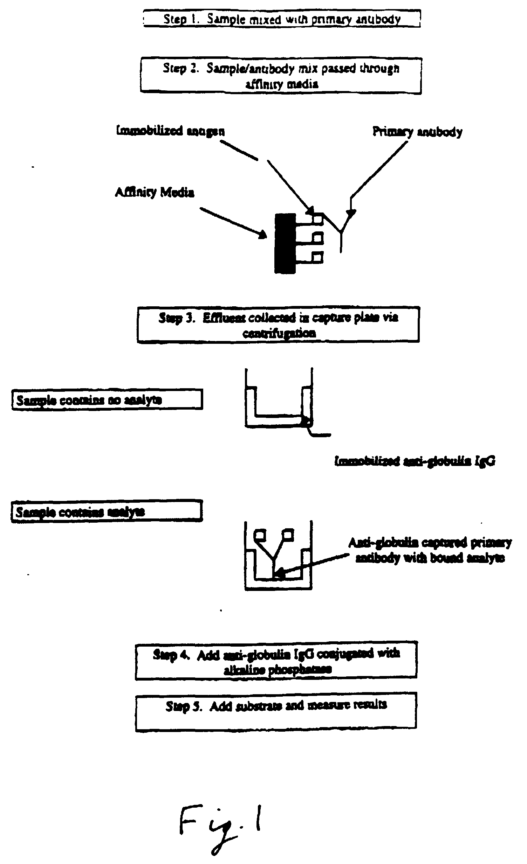

Aqueous standards containing the test substance, RH2485, were incubated with the first antibody, rabbit polyclonal antibody (anti-RH2651 IgG, THACO Research, Ltd.), at room temperature (25°C) for 15 minutes to allow for the formation of the first antibody, antibody-antigen complexes in solution. A 50-100 µL aliquot of this mixture was transferred to wells of the microfilter plate containing the antigen (RH2651) coated beads and incubated with shaking on an orbital shaker at room temperature for 30 minutes. Free or excess the first antibody, antibody (antibody not bound to antigen) was captured by the antigen-coated beads. The solution phase the first antibody, antibody-antigen complexes passed through the beads and were removed by centrifugation and collected in the capture plate containing immobilized goat, anti-rabbit IgG. The capture plate was incubated at room temperature for one hour then washed with the wash buffer. Reporter antibody (goat, anti-rabbit IgG-alkaline phosphatase conjugate, Pierce Chemical Co.) was added and incubated at room temperature for 30 minutes. After a final wash, substrate (*para*-Nitrophenylphosphate (PNPP), Pierce Chemical Co.) was added. After approximately 30 minutes, the absorbance was read at 410 nm using the MRX Microtiter® plate reader (Dynex Technologies, Inc.). A schematic of the method of the Example is presented in Figure 1.

As depicted in Figure 1, the test sample is mixed with the first antibody, antibody in step 1. In step 2, the mixture obtained in step 1 is passed through affinity media containing immobilized antigen. Thus, in step 2 excess or free the first antibody, antibody is captured by the immobilized antigen. In step 3, effluent is collected in a capture plate by centrifugation. The capture plate contains immobilized anti-globulin IgG. If the test sample contains an antigen for which the first antibody has specific affinity, the first antibody and antigen will form a complex and the anti-globulin IgG in the capture plate will capture this first antibody-antigen complex. In step 4, anti-globulin IgG conjugated with alkaline phosphatase is added. In step 5, the alkaline phosphatase substrate is added and the results are measured.

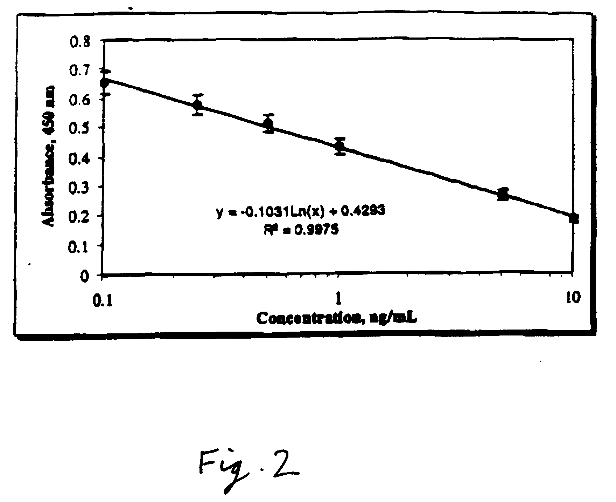

The results of the microfilter method of analysis are illustrated in Figure 2. Figure 2 is a standard curve established for RH2485 using the microfilter format. Error bars are the standard error of the mean from 11 replicate analyses. The microfilter assay for RH2485 exhibits a linear dynamic range over 3 log units of 1 ppb to 2 ppm with a positive slope.

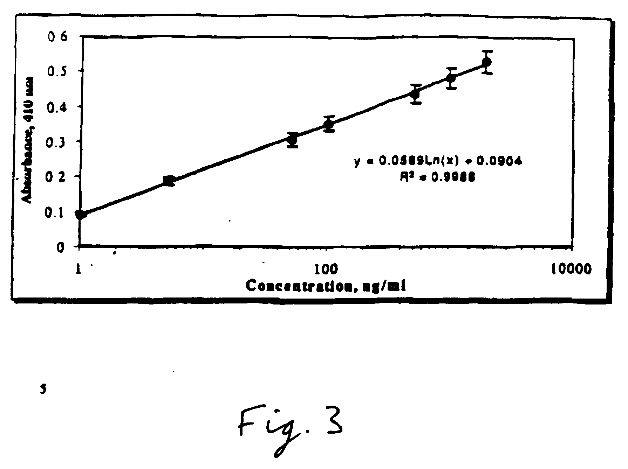

In contrast, a typical competitive ELISA for RH2485 has a narrower linear dynamic range of 2 log units from approximately 0.1 ppb to 10 ppb with a negative slope. Figure 3 depicts a standard curve established for RH2485 using a conventional standard competitive type capture ELISA. In a laboratory setting, the primary advantage of the wider linear range obtained by use of the present invention is that with true unknowns, fewer samples will fall outside the limits of the curve and consequently, the sample throughput will increase.

The resulting positive slope of Figure 2 can be explained mechanistically. A sample containing antigen is mixed with the first antibody, antibody having affinity for said antigen. Complexes formed by the first antibody and antigen are separated from any unbound first antibody by the antigen-bound affinity media. The solution phase first antibody-antigen complexes are captured by anti-globulin antibodies immobilized in wells of the capture plate. The presence of the captured first antibody-antigen complex is then probed with an anti-globulin-enzyme conjugate and visualized by substrate conversion to product. Thus, the more antigen present in the sample, the greater the intensity of the signal, resulting in a positive dose response.

The microfilter assay of the present example takes less than 3 hours to perform. The use of the microfilter format has certain intrinsic advantages to enhance overall assay performance. Since the initial first antibody-antigen reaction is occurring in the solution phase, equilibrium can be reached very fast, within 15 minutes. In contrast, in a typical ELISA, a monolayer of antigen is immobilized on a solid support and presents a limited surface area for the phase transfer process. With the antigen coated beads of the present invention, the surface area for the phase transfer is greatly increased and therefore the opportunity for contact between the antibody and the antigen is increased. In addition, the filtering process, which includes passing the antibody through the beads, increases the phase contact between the solution phase and the solid phase. The net effect of this modification is to improve the rate of the phase transfer process over standard solid phase ELISA techniques.

The rate-limiting step in standard competitive ELISA techniques is the phase transfer process. Typically the antigen or antibody is immobilized as a monolayer on a solid support such as a 96-well polystyrene plate. The analyte in solution is added to the wells ad incubated for sufficient time for the solution phase analyte to be captured by the solid phase antibody or antigen.

The invention described herein takes advantage of existing immunoassay components for specific test substances coupled with commercially available disposable 96-well microfilter plate technology to create a self contained sample preparation and analysis system. Using commercially available 96-well microfilter plates filled with antigen coated polystyrene beads, the surface area available for phase transfer is increased by the cube of the radius of the particles times the number of particles. Moreover, the analyte of interest or antigen is directly measured thereby producing a positive slope rather than the counterintuitive negative slope of conventional ELISA assays. Finally, the linear rage for the analyte RH2485 was increased by one order of magnitude when the novel microfilter ELISA technique of the present invention was used. These advantages should help ELISA technology become more widely accepted as a routine method for performing chemical and biological analyses.

Finally, the system of the present invention has the further advantage of having the capability of being fully automated. In a fully automated format, hundreds of samples may be analyzed per hour leaving the more expensive, time-consuming analyses such as HPLC, GC-MS and HPLC-MS to be used as confirmatory tests. The anticipated benefits of the high throughput analytical system of the present invention include more rapid sample analysis and greatly increased laboratory throughput with a commensurate reduction in analytical costs.

Other embodiments and uses of the invention will be apparent to those skilled in the art from consideration of the specification and practice of the invention disclosed herein. Thus, the invention includes a process for processing or preparing a product characterised in that a sample of the product or an intermediate used in its preparation is subjected to a method of the invention, the end product of the process optionally being a food material or a beverage material, for example drinking water. All references cited herein, for whatever reason, including U.S. provisional patent application entitled High Throughput ELISA System for Surface Water and Ground Water Analysis, serial number 60/077,895 (filed March 13, 1998), are specifically incorporated by reference. The specification and example should be considered exemplary only. As will be easily understood by those of ordinary skill in the art, variations and modifications of each of the disclosed embodiments can be easily made within the scope of this invention.

## Claims

1. A method for quantitating an antigen in a sample comprising the steps of:
contacting the sample with a first antibody having binding affinity to the antigen to form antigen-antibody complexes;
contacting uncomplexed antibody with excess antigen immobilized to a solid support to form immobilized antigen-antibody complexes;
capturing unbound antigen-antibody complexes by contacting said antigen-antibody complexes to an immobilized second antibody having binding affinity specific for the first antibody; and
quantitating the amount of antigen in the sample.

2. The method of claim 1 wherein the antigen is an agrochemical, a pesticide, a fungicide, a carcinogen, a biological antigen or a chemical antigen.

3. The method of claim 1 or claim 2 wherein the sample is a liquid, a solid or a liquid/solid suspension and/or is selected from the group consisting of ground water, a beverage, a dairy product, drinking water, a food sample and a biological sample.

4. The method of any of claims 1 to 3 wherein the first antibody, the second antibody or both antibodies are polyclonal antibodies or monoclonal antibodies.

5. The method of any of claims 1 to 4 wherein the solid support comprises polystyrene beads coated with the antigen or a microfilter, multiwell plate containing an antigen-conjugated matrix.

6. The method of any of claims 1 to 5 which further comprises labeling the antigen-antibody complexes with a detectable label, the detectable label optionally comprising an enzyme, a colored dye, a fluorescent material, a chemiluminescent material, a bioluminescent material or a radioactive isotope.

7. The method of claim 6 wherein the detectable label is conjugated with a material having binding affinity to the captured antigen-antibody complex, the material optionally being selected from the group consisting of one or more substances with antibody-antigen binding affinity, protein-ligand binding affinity or avidin-biotin binding affinity.

8. The method of claim 6 or claim 7 wherein quantitating is performed by effecting a measurable change in the label, and optionally wherein the measurable change is effected by irradiation with UV or visible light, fluorescent light, electrical wave form or by introduction of a substrate that interacts with the detectable label and/or the measurable change is fluorescence, luminescence, or ultraviolet absorbance.

9. The method of any of claims 1 to 7 wherein quantitating is performed by ultraviolet-visible light spectrophotometry, fluorometry or gamma counting.

10. The method of any of claims 1 to 9 further comprising separating bound antigen-antibody complexes from unbound antigen-antibody complexes, the separating optionally being performed by centrifugation, vacuum filtration, washing, or mixtures or combinations thereof.

11. A process for processing or preparing a product characterised in that a sample of the product or an intermediate used in its preparation is subjected to a method of any of claims 1 to 10, the end product of the process optionally being a food material or a beverage material, for example drinking water.

12. A kit for quantitating an antigen in a sample comprising:
a plurality of first antibodies having binding affinity to the antigen;
a plurality of polystyrene beads conjugated with the antigen;
a microfilter plate containing said plurality of polystyrene beads;
a capture plate containing a plurality of immobilized second antibodies, said second antibodies specific for said first antibody;
a label that identifies antibody-antigen complexes; and
a substrate that produces a measurable change in the presence, detection or quantity of said label,
the kit optionally further including the feature(s) recited in one or more of claims 2 and 4 and the optional portion of claim 6.
